(19) Europäisches Patentamt
European Patent Office
Office européen des brevets

(11) Veröffentlichungsnummer: **0 098 520**
**B1**

(12) **EUROPÄISCHE PATENTSCHRIFT**

(45) Veröffentlichungstag der Patentschrift: **29.01.86**

(51) Int. Cl.⁴: **A 61 K 37/02**, C 07 K 5/06, C 07 K 1/06

(21) Anmeldenummer: **83106342.5**

(22) Anmeldetag: **29.06.83**

(54) **L-Alanyl-D-Isoglutaminyl-adamantylamid, Verfahren zu seiner Herstellung und pharmazeutische Mittel mit dieser Verbindung.**

(30) Priorität: **29.06.82 CS 4908/82**

(43) Veröffentlichungstag der Anmeldung: **18.01.84 Patentblatt 84/3**

(45) Bekanntmachung des Hinweises auf die Patenterteilung: **29.01.86 Patentblatt 86/5**

(84) Benannte Vertragsstaaten: **AT BE CH DE FR GB IT LI SE**

(56) Entgegenhaltungen:
**FR - A - 2 482 957**

**CHEMICAL ABSTRACTS, Band 83, Nr. 13, 1975, Seite 605, Nr. 114859f, Columbus, Ohio, USA, W. KWAPISZEWSKI et al.: *"Synthesis of amino acid 1-adamantylamides"***
**CHEMICAL ABSTRACTS, Band 96, Nr. 9, 1. März 1982, Seite 659, Nr. 69439z, Columbus, Ohio, USA**
**CHEMICAL ABSTRACTS, Band 87, 1977, Seite 626, Nr. 118075c, Columbus, Ohio, USA**

(73) Patentinhaber: **SPOFA Spojené Podniky Pro Zdravotnickou Vyrobu, Husinecká 11 a, Prag 3 (CS)**

(72) Erfinder: **Flegel, Martin, Dr., Praha 2, Lumirova 5 (CS)**
Erfinder: **Krojidlo, Milan, Praha 8, Na Strelnici 555/B (CS)**
Erfinder: **Kolinsky, Jiri, Dipl. Ing., Praha 7, Nad Kralovskov oborov 11 (CS)**
Erfinder: **Masek, Karel, Dr., Praha 5, Xaveriova 2735 (CS)**
Erfinder: **Seifert, Jaroslav, Dr., Praha 6, U Ladronky 23 (CS)**

(74) Vertreter: **Patentanwälte Beetz sen. - Beetz jun. Timpe - Siegfried - Schmitt-Fumian, Steinsdorfstrasse 10, D-8000 München 22 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 098 520 B1**

ACTORUM AG

## Beschreibung

Die Erfindung betrifft L-Alanyl-D-isoglutaminyladamantylamid der Formel

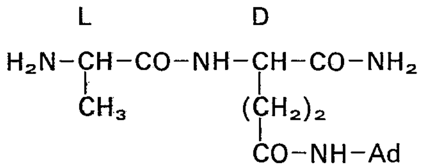

(I)

worin Ad den in dessen 1-Stellung gebundenen Adamantanrest bedeutet,
Verfahren zur Herstellung dieser Verbindung sowie entsprechende pharmazeutische Mittel.

Das erfindungsgemässe neue Dipeptidderivat, das im folgenden kurz als ADP bezeichnet ist, besitzt eine ausgeprägte immunoadjuvante und immunostimulierende Wirksamkeit.

Bekanntermassen stehen bis jetzt nur sehr wenige Substanzen zur Verfügung, die imstande sind, die Abwehrkraft des Organismus durch Stimulierung der Immunprozesse zu steigern. Hierzu wurden bisher zum Beispiel Bakterienprodukte oder Zellwandprodukte verwendet. Ihre Nachteile lagen in den nicht standardisierbaren Wirkungen sowie in gravierenden Nebenwirkungen bei der klinischen Anwendung. Eine ähnliche Situation besteht auf dem Gebiet der Stoffe mit immunoadjuvanter Wirksamkeit, wo wiederum hauptsächlich Bakterienprodukte aus Mycobacterium tuberculosis, und zwar häufig in Kombination mit Mineralölen, angewandt werden.

Ein bedeutender Fortschritt wurde durch die Gewinnung einiger wirksamer Untereinheiten von Bakterienwänden erzielt, die zunächst enzymatisch und später auch synthetisch durchgeführt wurde (F. Ellouz *et al.*, Biochem. Biophys. Res. Commun. *59* (1974) 1317). Die umfangreichsten experimentellen Erfahrungen wurden mit Muramyldipeptid (N-Acetylmuramyl-L-alanyl-D-isoglutamin, MDP) und mit einer Reihe seiner Analoga erhalten (L. Chedid *et al.*, Progr. Allergy *25*, 63, Karger, Basel 1978; C. Merser *et al.*, Biochem. Biophys. Res. Commun. *66* (1975) 1316). Bei diesen Analoga liegen unterschiedliche biologische Wirkungen vor; bei der Untersuchung verschiedener Strukturen wurde im Lauf der Zeit festgestellt, dass für die adjuvante und immunostimulierende Wirkung offenbar der peptidische Molekülteil von entscheidender Bedeutung ist und die Wirksamkeit mehr oder weniger von der Art des Zuckerrests im Molekül unabhängig ist (L. Chedid *et al.*, loc. cit.; C. Merser *et al.*, loc. cit.; K. Mašek *et al.*, Experientia *35* (1979) 1397; S. Kotani *et al.*, Biken's J. *18* (1975) 105; A. Hasegawa *et al.*, Agric. Biol. Chem. *42*, Nr. 11 (1978) 2187).

Es wurde ferner bereits beschrieben, dass die Wirkung von MDP und einigen seiner Analoga höher und stabiler ist, wenn diese in einem Mineralölmedium oder in an liposomale Strukturen gebundener Form verabreicht werden (J. Freud, K. McDermot, Proc. Soc. Expt. Biol. Med. *49* (1942) 548; K. Mašek *et al.*, Experientia *34* (1978) 1363). Sofern die bisher geprüften synthetisch hergestellten Substanzen biologisch wirksam waren, waren sie gleichzeitig jeweils mehr oder weniger pyrogen, was der Hauptgrund war, weshalb sie in der klinischen Praxis keine Anwendung fanden. In diesem Zusammenhang wurde sogar vermutet, dass die adjuvanten Wirkungen direkt mit der Pyrogenität zusammenhängen (S. Kotani *et al.*, Birken's J. *19* (1976) 9).

Eine sehr bedeutende Rolle spielt bei der biologischen Wirksamkeit offensichtlich die gesamte Lipophilie des Moleküls, was durch Herstellung von Analoga, die einen an die Hydroxylgruppe in 5-Stellung der Saccharidkomponente gebundenen höheren Alkylrest enthielten, bestätigt wurde (S. Kotani *et al.*, Biken's J. *19* (1976) 9). Eine ähnliche Wirkung wurde bei Einführung einer lipophilen Kette an der γ-Carboxylgruppe des Isoglutamins beobachtet (K. Mašek *et al.*, Experientia *35* (1979) 1397).

Im Rahmen der Erfindung wurde von der Feststellung der bedeutenden Rolle des Dipeptidteils (L-Alanyl-D-isoglutamin) bei der adjuvanten und immunostimulierenden Wirksamkeit sowie davon ausgegangen, dass die lipophile Struktur für die gesamte pharmakologische Wirksamkeit von Bedeutung ist. Durch Einführung des stark lipophilen, an die γ-Carboxylgruppe der Isoglutaminsäure amidartig gebundenen Adamantanrestes wurde nun das erfindungsgemässe L-Alanyl-D-isoglutaminyladamantylamid (ADP) der oben angeführten Formel erhalten, das in völlig überraschender Weise eine bedeutende biologische Wirksamkeit ohne unerwünschte Nebenwirkungen wie Pyrogenität aufweist und zugleich leicht synthetisch zugänglich ist.

Die erfindungsgemässe Verbindung ADP ist nach in der Peptidchemie üblichen Verfahren zugänglich, insbesondere durch Kondensation des geschützten L-Alanyl-D-isoglutamins mit 1-Aminoadamantan.

Nach einer bevorzugten Verfahrensweise wird beispielsweise ein Dipeptidderivat der allgemeinen Formel II

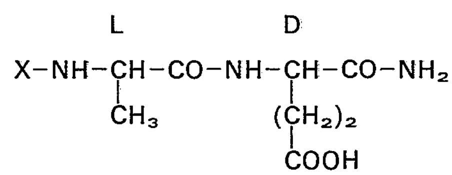

(II)

in der X eine durch saure Hydrolyse oder Hydrogenolyse abspaltbare Schutzgruppe bedeutet, vorzugsweise t-Butyloxycarbonyl, Benzyloxycarbonyl oder o-Nitrophenylthio, mit 1-Aminoadamantan umgesetzt, wonach die Schutzgruppe abgespalten wird.

Das folgende Beispiel erläutert das erfindungsgemässe Verfahren, wobei BOC t-Butyloxycarbonyl, Ala Alanyl und Iglm Isoglutamin bedeuten.

*Beispiel*

t-Butyloxycarbonyl-L-alanyl-D-isoglutamin

Zu einer Lösung von 19,6 g BOC-Ala und 12,3 g N-Hydroxybenzotriazol in 280 ml Dioxan wurden

bei −10° C 20,3 g Dicyclohexylcarbodiimid zugegeben. Nach 60 min Rühren bei Raumtemperatur wurde filtriert und das Filtrat zu einer Lösung von 13,3 g D-Isoglutamin in 60 ml Wasser zugegeben, worauf über Nacht weiter gerührt wurde. Danach wurde das Reaktionsgemisch filtriert und das Filtrat zur Trockne eingedampft. Der Rückstand wurde aus einem Ethylacetat-Ether-Gemisch umkristallisiert. Ausbeute des Produkts 30,9 g (83% d. Th.); F. 98° C; $[\alpha]_D^{20}$ −8,2 (c-1, Methanol).

t-Butyloxycarbonyl-L-alanyl-D-isoglutaminyl-adamantylamid

Aus 1,9 g 1-Aminoadamantanhydrochlorid wurde durch Einwirkung von 2N Natriumhydroxidlösung die Base freigesetzt, die mit Chloroform extrahiert wurde. Durch Eindampfen wurden 1,4 g (9 mmol) Produkt erhalten, das in 30 ml Dimethylformamid gelöst wurde. Die Lösung wurde auf −10° C abgekühlt und mit einer Lösung von 2,1 g (10 mmol) Dicyclohexylcarbodiimid in 10 ml Dimethylformamid versetzt. Nach 5 min wurde eine Lösung von 1,6 g (5 mmol) BOC-L-Ala-D-Iglm, 1,3 g N-Hydroxybenzotriazol, 0,7 ml Triethylamin und 5 ml Pyridin in 20 ml Dimethylformamid zugegeben. Nach Rühren des Reaktionsgemischs bei Raumtemperatur über Nacht wurde das Dimethylformamid abgedampft und der Rückstand in 100 ml Ethylacetat gelöst, worauf die Lösung wiederholt mit 0,1N Salzsäure und 5%iger Natriumhydrogencarbonatlösung extrahiert und danach getrocknet und eingedampft wurde. Durch Fällen der methanolischen Lösung des Rückstands mit Wasser wurden 1,28 g (56% d. Th.) des gewünschten Produkts erhalten, dessen Elementar- und Aminosäureanalyse der Berechnung entsprachen.

L-Alanyl-D-isoglutaminyl-adamantylamid

Aus dem vorstehenden Produkt wurde durch Einwirkung einer 40%igen Trifluoressigsäurelösung in Methylenchlorid die t-Butyloxycarbonylgruppe abgespalten. Nach 60 min Stehen bei Raumtemperatur wurde die Lösung eingedampft und der Rückstand mit Ether versetzt. Durch Absaugen wurden 0,9 g Produkt (in Form des Trifluoracetats) erhalten, das sofort in 5 ml Methanol gelöst, auf eine Säule eines Anionenaustauschers in der OH-Form aufgegeben und mit Methanol eluiert wurde. Durch Eindampfen des Eluats wurden 0,8 g (83% d. Th.) eines schaumartigen Produkts erhalten.

Aminosäurenanalyse: Ala 1,01, Glu 0,97.

Die Reinheit des Produkts wurde ferner durch Hochdruck-Flüssigkeitschromatographie (HPLC) und Papierelektrophorese in Puffern mit pH 2,5 und 5,7 nachgewiesen.

Das L-Alanyl-D-isoglutaminyl-adamantylamid wurde unter folgenden Bedingungen analysiert: Als stationäre Phase wurde ein modifiziertes Kieselgel mit lipophilen Gruppen (Kolonne 15× 0,3 cm) verwendet; die mobile Phase enthielt 60 bis 80 Vol.-% eines organischen Modifizierungsmittels, vorzugsweise Methanol, und 20 bis 40 Vol.-% 0,2%ige wässerige Trifluoressigsäure. Die Messung erfolgte mit einem UV-Detektor bei 210 nm. Der Durchsatz an der mobilen Phase betrug 20 bis 30 ml/h. Für präparative Zwecke wurden die gleiche stationäre und mobile Phase und eine grösser dimensionierte Kolonne (30×2,5 cm) verwendet.

Die Reinheit des Produkts wurde ferner durch Dünnschichtchromatographie an Kieselgel mit den Systemen n-Butanol – Essigsäure – Wasser (4:1:1) und Chloroform – Methanol – Essigsäure – Wasser (40:20:10:5) nachgewiesen.

Aus 0,5 g Rohprodukt wurden durch Reinigung mit HPLC 0,3 g Substanz in reiner Form erhalten (Gehalt etwa 95 Gew.-% nach der Peak-Fläche), die zu pharmakologischen Untersuchungen verwendet wurde.

Pharmakologische Eigenschaften des L-Alanyl-D-isoglutaminyl-adamantylamids

Test 1

Die immunoadjuvante Wirksamkeit wurde an weissen Meerschweinchen getestet, denen in die linke Hinterpfote 0,2 ml eines Gemischs aus Ovalbumin (2,5 mg) und L-Alanyl-D-isoglutaminyl-adamantylamid (ADP) in einer Menge von 100 µg in nicht vollständigem Freundschen Adjuvans (FIA) verabreicht wurden. Die Kontrollgruppe erhielt lediglich ein Gemisch aus dem nicht vollständigen Freundschen Adjuvans mit Ovalbumin. Die Wirksamkeit des geprüften Stoffs wurde dann mit der des bisher am stärksten wirkenden bekannten bakteriellen Adjuvans Mycobacterium tuberculosis verglichen. Der entsprechenden Tiergruppe wurde hierzu ein Gemisch verabreicht, das Ovalbumin mit dem kompletten Freundschen Adjuvans enthielt. Die Wirksamkeit der erfindungsgemässen Verbindung wurde ferner mit der Wirksamkeit von N-Acetylmuramyl-L-alanyl-D-isoglutamin (MDP) verglichen.

Drei Wochen nach der Verabreichung der getesteten Stoffe wurde dann bei den Meerschweinchen die Hautreaktion auf Verabreichung von 10 und 20 µg Ovalbumin beobachtet. Die als Grösse der Hautreaktionsfläche in mm ausgedrückten Ergebnisse der Messungen sind in Tabelle 1 aufgeführt.

*(Tabelle auf der nächsten Seite)*

Aus den Ergebnissen von Tabelle 1 ist ersichtlich, dass die Verabreichung des erfindungsgemässen Derivats zu einer Steigerung der Verzögerung der Überempfindlichkeit bei Meerschweinchen gegenüber Ovalbumin führt und die Wirkung bei der angewandten Dosis von 100 µg mit der von MDP sowie mit der von FCA vergleichbar ist.

Test 2

Zur Untersuchung der DNA-Biosynthese in vivo wird meistens markiertes Thymidin verwendet. Dieses vorgebildete Pyrimidindesoxynucleosid wird durch eine Reihe von Phosphorylierungsreaktionen zu Desoxythymidintriphosphat (DTTP) phosphoryliert, das den unmittelbaren Vorläufer für die DNA-Synthese im Zellkern darstellt. Ein erhöhter Einbau des markierten Thymidins in der

*Tabelle 1*

Induktion der verzögerten Überempfindlichkeit gegenüber Ovalbumin bei Meerschweinchen mit synthetischem ADP

| Geprüfte Substanz | Dosis | Hautreaktionsfläche nach 24 h (mm) | |
|---|---|---|---|
| | | Verabreichung von 10 µg Ovalbumin | Verabreichung von 20 µg Ovalbumin |
| FIA | — | 1,0 ±0,48 | 3,39±0,22 |
| FCA | 1 mg | 3,71±0,23* | 5,73±0,33* |
| MDP | 100 µg | 2,99±0,23* | 6,07±0,21* |
| ADP | 100 µg | 2,70±0,21* | 5,82±0,27* |

Die angegebenen Zahlenwerte sind Mittelwerte aus zwei Versuchen mit 16 Tieren.

* statistisch signifikante Unterschiede (p>0,05).
FIA = unvollständiges Freundsches Adjuvans (Bayol + Arlacel 4:1)
FCA = vollständiges Freundsches Adjuvans (Bayol + Arlacel + 1 mg Mycobacterium tuberculosis)
MDP = N-Acetyl-muramyl-L-alanyl-D-isoglutamin
ADP = L-Alanyl-D-isoglutaminyl-adamantylamid

DNA entspricht einer Aktivierung der Biosynthese, die der Zellteilung vorausgeht.

Dieser Test ist einer der empfindlichsten und zuverlässigsten, mit dem sich die immunostimulierenden Wirkungen von Stoffen und ihre Wirkung auf die Zellteilung messen lassen.

Männlichen Ratten (Stamm Wistar, Körpergewicht 150 bis 160 g) wurden die geprüften Stoffe in äquimolaren Konzentrationen verabreicht, d.h. MDP in einer Dosis von 1,00 mg/kg und ADP in einer Gabe von 0,74 mg/kg. Die Tiere wurden nach 16 h getötet; 2 h vor dem Töten wurde ihnen intraperitoneal markiertes Thymidin (Methyl-$^3$H-Thymidin) in einer Dosis von $7,4 \cdot 10^{-15} s^{-1}$ (20 µCi) pro Tier in 0,3 ml physiologischer Kochsalzlösung (spezifische Aktivität $7,4 \cdot 10^{-15} s^{-1}$/mmol (20 Ci/mmol)) verabreicht.

Nach dem Einfrieren wurden die gefrorenen Organe entnommen und rasch in 0,2N Perchlorsäure homogenisiert. Nach wiederholtem Waschen des Sediments mit 0,2N Perchlorsäure wurde durch alkalische Hydrolyse die RNA abgetrennt. Aus dem Protein-DNA-Präzipitat wurden die Nucleotidkomponenten durch Hydrolyse in 1N Perchlorsäure freigesetzt. Die flüssige Phase wurde nach Neutralisation zur Trockne eingedampft. Durch eine weitere saure Hydrolyse in konz. Perchlorsäure wurden die DNA-Nucleotide in Basen überführt. Das Thymin wurde danach in chromatographisch reiner Form durch wiederholte Papierchromatographie isoliert; seine Radioaktivität wurde mit einem Szintillationsspektrometer gemessen. Die Versuchsergebnisse sind in Tabelle 2 aufgeführt.

*Tabelle 2*

Einbau von Methyl-$^3$H-Thymidin bei der DNA-Synthese in Leber, Niere, Thymus und Milz von Ratten nach Verabreichung von MDP und ADP

| | cpm/µl DNA-Thymin | | | |
|---|---|---|---|---|
| | Leber | Niere | Thymus | Milz |
| Kontrolle | 1750 | 1750 | 940 | 11 150 |
| MDP (Muramyldipeptid) | 4100 | 2350 | 1400 | 10 900 |
| ADP (Adamantyldipeptid) | 2950 | 2200 | 1550 | 16 500 |

Aus den Ergebnissen von Tabelle 2 ist ersichtlich, dass bereits eine einmalige Verabreichung von ADP in einer Dosis von 0,75 mg/kg eine ausgeprägte Wirkung auf den Einbau von Methyl-$^3$H-Thymidin bei der DNA-Synthese im Thymus und in der Milz ausübt. Beide Organe beteiligen sich in erheblichem Masse an der Ausbildung der Tumor- und Zellimmunität. Aus den Ergebnissen der Tabelle 2 ist eine signifikante Erhöhung des Einbaus des Thymidins im Thymus zu ersehen, die etwa der im Falle von MDP entspricht, d.h. eines Präparats, bei dem diese Wirkung schon lange bekannt ist. Im

Falle der Milz ist festzustellen, dass, obgleich die Verabreichung von MDP hier keine erhöhte Ausnutzung des Thymidins hervorruft, ADP hierbei ebenfalls stimulierende Wirkungen aufweist, die sich durch einen erhöhten Einbau von $^3$H-Thymidin erweisen.

Die erhaltenen Ergebnisse über die Wirkung von MDP auf die Ausnutzung des Thymidins in der Milz stimmen gut mit den Angaben in der Literatur überein, aus denen hervorgeht, dass MDP keine Wirkung auf dieses Organ ausübt, auch nicht auf isolierte Zellen in vitro. Beim Vergleich der Wirksamkeit des erfindungsgemässen ADP mit der Vergleichssubstanz MDP erweist sich ADP als deutlich vorteilhafter, da es offensichtlich in ausgeprägter Weise auf beide Organe wirkt, die eine wichtige Rolle bei Immunreaktionen spielen.

Test 3

Die Mehrzahl der bisher synthetisierten MDP-Analoga besitzen ausser immunoadjuvanten und immunostimulierenden Wirkungen auch eine ausgesprochene pyrogene Wirksamkeit, was für die klinische Praxis einen erheblichen Nachteil darstellt; bis jetzt verhinderte diese Tatsache die klinische Anwendung einiger ansonsten sehr erfolgversprechender Substanzen.

In Experimenten an Kaninchen wurde deshalb die pyrogene Wirkung von ADP im Vergleich mit MDP bewertet. Kaninchen mit einem Körpergewicht von 1,8 bis 2,0 kg wurde ADP intravenös in einer Dosis von 100 µg/kg verabreicht; die Wirkungen wurden bei gleicher Dosis mit denen von MDP verglichen. Den Kontrolltieren, bei denen die Basaltemperatur gemessen wurde, wurde dann ein gleiches Volumen physiologischer Kochsalzlösung (0,2 ml) injiziert. Die Temperatur der Kaninchen wurde 6 h lang rektal mit einem Thermistorthermometer in 1-h-Intervallen gemessen; die erhaltenen Ergebnisse sind in Tabelle 3 zusammengefasst.

*Tabelle 3*

Pyrogene Wirkung von MDP und ADP bei Kaninchen

| Geprüfte Substanz | Dosis (µg/kg) | Temperaturänderung (°C) nach | | | | | |
|---|---|---|---|---|---|---|---|
| | | 1 h | 2 h | 3 h | 4 h | 5 h | 6 h |
| Kontrollen (physiol. Lösung) | — | −0,1 | +0,2 | −0,2 | −0,1 | 0 | +0,1 |
| ADP | 100 | +0,3 | +0,4 | +0,4 | +0,4 | +0,3 | +0,35 |
| MDP | 100 | +0,8 | +1,7 | +1,8 | +1,3 | +0,7 | +0,4 |

Die in Tabelle 3 angeführten Werte sind Mittelwerte von 5 Tieren pro Gruppe.

Aus den Ergebnissen der Tabelle 3 geht hervor, dass die erfindungsgemässe Verbindung ADP in Dosen, die für die klinische Anwendung in Betracht kommen, keine pyrogene Wirksamkeit besitzt. Bei der gleichen Dosis von 100 µg/kg ist die Vergleichssubstanz MDP bereits ausgeprägt pyrogen. Der Umstand, dass ADP bei vergleichbarer immunostimulierender und immunoadjuvanter Wirksamkeit keine pyrogene Wirkung hat, stellt einen ausserordentlichen, unerwarteten Vorteil dar und ermöglicht die klinische Anwendung.

Aufgrund der oben angeführten Ergebnisse der pharmakologischen Untersuchungen kann die erfindungsgemässe Verbindung als pharmakologisch günstig geeigneter Wirkstoff angesehen werden. Entsprechende pharmazeutische Mittel, zweckmässig in Form eines Präparats mit üblichen Hilfs- und/oder Trägerstoffen, eignen sich z.B. zur Steigerung der Immunreaktionen des Organismus in der Human- und Veterinärmedizin und insbesondere zur Erhöhung der Widerstandsfähigkeit bei Zuständen, die mit einer verminderten Bildung von Antikörpern einhergehen.

Die erfindungsgemässen Mittel lassen sich demgemäss besonders als Immunostimulantien einsetzen.

**Patentansprüche**

1. L-Alanyl-D-isoglutaminyl-adamantylamid der Formel

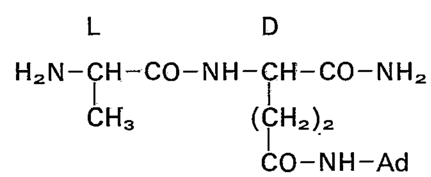

(I)

worin Ad den in 1-Stellung gebundenen Adamantanrest bedeutet.

2. t-Butyloxycarbonyl-L-alanyl-D-isoglutaminyl-adamantylamid als Zwischenprodukt zur Herstellung von L-Alanyl-D-isoglutaminyl-adamantylamid.

3. Benzyloxycarbonyl-L-alanyl-D-isoglutaminyl-adamantylamid als Zwischenprodukt zur Herstellung von L-Alanyl-D-isoglutaminyl-adamantylamid.

4. o-Nitrophenylthio-L-alanyl-D-isoglutaminyl-adamantylamid als Zwischenprodukt zur Herstellung von L-alanyl-D-isoglutaminyl-adamantylamid.

5. Verfahren zur Herstellung von L-Alanyl-D-isoglutaminyl-adamantylamid nach Anspruch 1,

gekennzeichnet durch Umsetzung eines Dipeptidderivats der allgemeinen Formel II

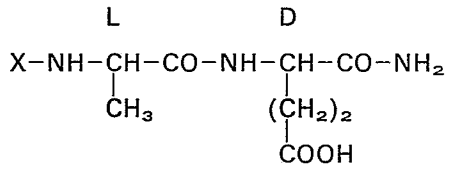
(II)
.

worin X eine durch saure Hydrolyse oder Hydrogenolyse abspaltbare Schutzgruppe bedeutet, mit 1-Aminoadamantan und anschliessende Abspaltung der Schutzgruppe.

6. Verfahren nach Anspruch 5, gekennzeichnet durch Verwendung einer Verbindung der Formel II mit X = t-Butyloxycarbonyl, Benzyloxycarbonyl oder o-Nitrophenylthio als Schutzgruppe.

7. Pharmazeutische Mittel, gekennzeichnet durch den Gehalt an einer Verbindung nach Anspruch 1, gegebenenfalls im Gemisch mit üblichen Hilfs- und/oder Trägerstoffen.

## Claims

1. L-Alanyl-D-isoglutaminyl-adamantylamide of the formula I

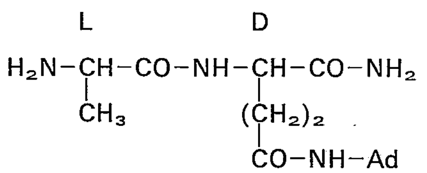
(I)

wherein Ad stands for an adamantane residue bound in position 1.

2. t-Butyloxycarbonyl-L-alanyl-D-isoglutaminyl-adamantylamide as an intermediate for the preparation of L-alanyl-D-isoglutaminyl-adamantylamide.

3. Benzyloxycarbonyloxy-L-alanyl-D-isoglutaminyl-adamantylamide as an intermediate for the preparation of L-alanyl-D-isoglutaminyl-adamantylamide.

4. o-Nitrophenylthio-L-alanyl-D-isoglutaminyl-adamantylamide as an intermediate for the preparation of L-alanyl-D-isoglutaminyl-adamantylamide.

5. A process for the preparation of L-alanyl-D-isoglutaminyl-adamantylamide of claim 1 comprising reacting a dipeptide derivative of the general formula II

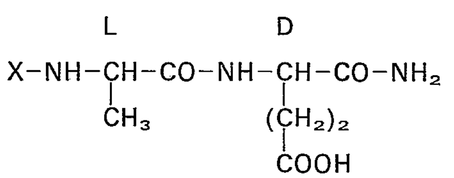
(II)

wherein X stands for a protective group removable by acidic hydrolysis or by hydrogenolysis, with 1-aminoadamantane and subsequent removal of the protective group.

6. A process of claim 5, characterized in that a compound of formula II with X being a t-butoxycarbonyl, benzyloxycarbonyl or o-nitrophenylthio group as the protective group is used.

7. Pharmaceutical compositions comprising a compound of claim 1, if required in a mixture with common auxiliaries and/or carriers.

## Revendications

1. L-alanyl-D-isoglutaminyl-adamantylamide de formule

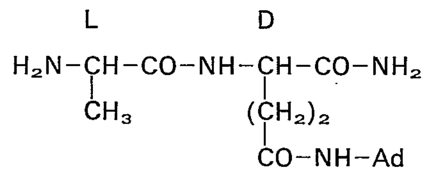
(I)

dans laquelle Ad désigne le radical adamantane fixé en position 1.

2. Tert-butyloxycarbonyl-L-alanyl-D-isoglutaminyladamantylamide, en tant que produit intermédiaire pour la préparation du L-alanyl-D-isoglutaminyl-adamantylamide.

3. Benzyloxycarbonyl-L-alanyl-D-isoglutaminyl-adamantylamide, en tant que produit intermédiaire pour la préparation du L-alanyl-D-isoglutaminyl-adamantylamide.

4. o-Nitrophénylthio-L-alanyl-D-isoglutaminyl-adamantylamide, en tant que produit intermédiaire pour la préparation du L-alanyl-D-isoglutaminyl-adamantylamide.

5. Procédé pour la préparation de L-alanyl-D-isoglutaminyl-adamantylamide selon la revendication 1, caractérisé par la réaction d'un dérivé dipeptidique de formule générale II

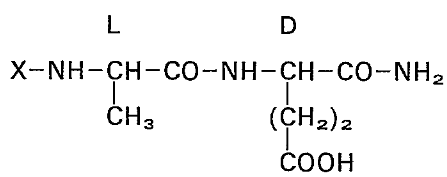
(II)

dans laquelle X désigne un groupe protecteur pouvant être dissocié par hydrolyse acide ou hydrogénolyse, sur le 1-amino-adamantane, suivie d'une dissociation du groupe protecteur.

6. Procédé selon la revendication 5, caractérisé par l'utilisation d'un composé de formule II avec X = tert-butyloxycarbonyle, benzyloxycarbonyle ou o-nitrophénylthio en tant que groupe protecteur.

7. Produits pharmaceutiques, caractérisés en ce qu'ils contiennent un composé selon la revendication 1, éventuellement en mélange avec des auxiliaires et/ou supports habituels.